# EUROPEAN PATENT APPLICATION

(11) **EP 3 545 913 A1**
(43) Date of publication of application: **02.10.2019**
(21) Application number: 19166424.2
(22) Date of filing: 29.03.2019
(51) Int. Cl.: A61F 2/56, A61F 2/68, A61F 2/58, A61F 2/70

(54) **IMPROVEMENTS IN OR RELATING TO PROSTHETIC LIMBS**

(30) Priority: 29.03.2018 GB 201805307
(71) Applicant: Open Bionics Ltd, Bristol BS34 8QZ (GB)
(72) Inventor: GIBBARD, Joel, Backwell, Bristol BS48 3LE (GB); PAYNE, Samantha, Backwell, Bristol BS48 3LE (GB); RAINES, Jonathan, Horfield, Bristol BS7 0BN (GB); MCVEIGH, Brian, St Werburghs, Bristol BS2 9UD (GB); QATTAN, Ahlam, Barnoldswick, Lancashire BB18 6AA (GB); WOOD, Steve, Swindon, Wiltshire SN2 1LT (GB)
(74) Representative: Bryers LLP

(57) **Abstract**

An actuator for a prosthetic digit is provided. The actuator comprises a driven, mobile tendon tensioner (70) to which a tendon (60) can be attached. The tensioner being movable along a shaft (72) in either direction so as to place tension on the tendon or to relax the tendon.

## Description

The present invention relates generally to prosthetic limbs and particularly, although not exclusively, to a hand for a robotic prosthetic arm.

A prosthesis is an artificial device that replaces a missing body part, which may be lost through trauma, disease, or congenital conditions. Prosthetics are intended to restore the normal functions of the missing body part.

The present invention seeks to provide improvements in or relating to prosthetic limbs.

An aspect of the present invention provides an actuator for a prosthetic digit, the actuator comprising a driven, mobile tendon tensioner to which a tendon can be attached, the tensioner being movable along a shaft in either direction so as to place tension on the tendon or to relax the tendon.

The digit may be a prosthetic finger or toe, which may form part of a prosthetic hand/foot, which in turn may form part of a prosthetic arm or leg.

The shaft may comprise a lead screw that can be rotated in either direction.

The actuator may comprise means for preventing rotation of the tensioner as it moves along the shaft, such as one or more guide rods.

In some embodiments the shaft can be rotated by a motor.

The actuator may further comprise a hitchhiker member to which a tendon is attached but which is not connected to the shaft.

The hitchhiker may prevent slack in the tendon.

The hitchhiker may prevent slack in the tendon as tensioner moves from a more tensioned position to a less tensioned position.

The present invention also provides an actuator block comprising one or more actuators as described herein.

The present invention also provides a prosthetic limb comprising one or more actuators as described herein.

The present invention also provides an actuator for a prosthetic digit, the actuator comprising a driven, mobile tendon tensioner, the tensioner being movable along a drive shaft that can be rotated in either direction by a motor so as to place tension on the tendon or the relax the tendon.

In some embodiments motors work through a gearbox to move drive nuts backwards and forwards along lead screws.

Each tendon may be connected to a drive nut (e.g. the nut has two holes and the tendon is threaded through the holes) so the tendon moves with the nut. The nut may be threaded onto a lead screw. Running parallel to and either side of the lead screw may be a guide rod. The nut may be prevented from rotating by the guide rods, along which the nut can slide. This means that when the lead screw is rotated by the motor, depending on which way the screw is rotated, the nut moves towards a proximal end or a distal end of the block.

In some embodiments when the nut moves towards the proximal end this pulls on the tendon and the finger will flex. When the nut moves towards the distal end this relaxes the tendon and the springs cause the finger to straighten (i.e. the motors do not power finger extension).

In some embodiments, in normal operation the tendon will move backwards and forwards with the nut, with no slack in the tendon. However, if extension of the finger is impeded (e.g. if the user tries to straighten the finger but cannot, for example because they are holding it closed, or if the fingers are prevented from straightening as they are held on a surface) this can create a problem. For example, if the finger is fully flexed, with the nut at or towards the proximal ends, and then the user tries to straighten the finger (but cannot) there is no tendon tension; this causes slack in the tendon.

To solve this problem some embodiments of the present invention may use "hitchhikers". The hitchhikers may not attached to the lead screw, but they can slide along the guide rods. The tendon may also be looped through the hitchhikers.

In normal operation the hitchhiker is pushed proximally by the nut and is pulled by the tendon distally, so that it follows the nut (i.e. it "hitches a ride"). For example as the finger is bent the nut is moved proximally by the motor and the hitchhiker is pushed proximally by the nut. As the finger straightens the nut is moved distally by the motor and the hitchhiker is pulled by the tendon (and is allowed to move along behind the nut).

In the case where the finger cannot straighten the hitchhiker stays where it is even though the nut is being moved by the motor. There is no tendon tension so the hitchhiker remains stationary, preventing slack in the tendon. When the finger is eventually released there is tension back on the tendon and the hitchhiker is pulled back onto the proximal side of the nut.

A further aspect of the present invention provides a prosthetic hand having one or more digits, the hand comprising one or more actuators for actuating digits, and a main control PCB.

In some embodiments the hand has four fingers and a thumb.

The present invention also provides a prosthetic finger/thumb having phalanges which can be caused to flex with respect to each other via retraction of a tendon-like member, the finger having, on its dorsal sides, an extension spring for causing the finger to extend when the tendon is relaxed.

The present invention also provides a prosthetic finger/thumb having a tendon for causing flex, the tendon terminating in a finger tip region, the finger having means for adjusting the tension in the tendon.

A prosthetic hand comprising one or more fingers/digits as described herein is also provided.

A present invention also provides a robotic prosthetic arm, comprising a ventilated outer frame and a ventilated inner liner.

The present invention also provides a prosthesis, such as a prosthetic arm, comprising an outer frame and an inner socket, the outer frame having attachment points for receiving a removable cover.

According to a further aspect of the present invention there is provided a prosthetic arm comprising an outer frame and an inner socket, the outer frame having a plurality of airflow openings and the inner socket having a plurality of airflow openings.

In some embodiments the present invention provides a transradial prosthesis - an artificial limb that replaces an arm missing below the elbow. In other embodiments the present invention provides a transhumeral prosthesis - a prosthetic lower and upper arm, including a prosthetic elbow.

In some embodiments the present invention provides or relates to a myoelectric prosthesis, which uses the electrical tension generated every time a muscle contracts, as information.

The outer frame may have an open core lattice structure. This provides strength whilst at the same time inherently providing ventilation.

The socket may have a plurality of longitudinal flutes. The socket may, therefore, have a generally cylindrical and "concertina-like" configuration. This allows, for example, the socket to be expandable and compressible. Vent hols may be formed in the flutes.

The socket may be flexible. The flexibility may be provided by material choice and/or structural form.

In some embodiments the frame can be tensioned on to or around the socket. The frame may be relatively rigid and the socket may be relatively flexible so that tightening of the frame can tension the socket to provide a good fit onto the patient.

The frame may comprise attachment points for a removable cover. In some embodiments magnetic attachment means is provided.

Different aspects and embodiments of the invention may be used separately or together.

Further particular and preferred aspects of the present invention are set out in the accompanying independent and dependent claims. Features of the dependent claims may be combined with the features of the independent claims as appropriate, and in combination other than those explicitly set out in the claims.

### Brief Description of the Drawings

The present invention will now be more particularly described, by way of example, with reference to the accompanying drawings, in which:
Figure 1 - exploded view of a prosthetic arm formed according to an embodiment.
Figure 2 - Palmar view of the tendon layout for a three-motor variant of an actuator block.
Figure 3 - Palmar view of the tendon layout for a four-motor variant of an actuator block.
Figure 4 - construction of a finger.
Figure 5 - simplified cross-section of the finger, showing the tendon path (highlighted in blue) and cross-section of a flexed finger, showing the tendon path (highlighted in blue).
Figure 6 - Tendon wrapping and clamping.
Figure 7 - Lateral cross section of the palm chassis and thumb ligament cover showing the path of the thumb tendon (highlighted).
Figure 8 - Exploded view of the palm.

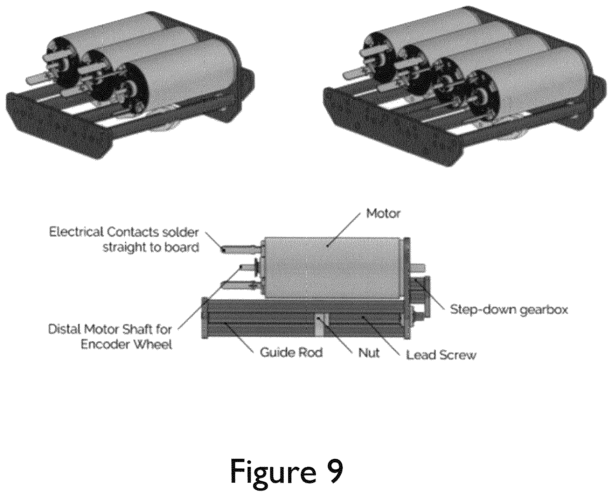

Figure 9 - Left: Three motor variant. Right: Four motor variant. Centre: Annotated lateral view.
Figure 10A - Normal Extension and Impeded Extension in a Hitchhiker System.
Figure 10B - Normal Extension and Impeded Extension in a System without Hitchhikers.
Figure 11 - Prosthetic Arm.
Figure 12 - Prosthetic Arm Exploded View.

### Definitions

Palmar - the side of something closest to the palm.
Axial Plane - the plane defined by a normal running axial to the object in question. If no object is specified, it should be assumed the term is being used in the broader anatomical way where the axial vector runs from head to foot through the body.
PCB - Printed Circuit Board

Example embodiments are described below in sufficient detail to enable those of ordinary skill in the art to embody and implement the systems and processes herein described. It is important to understand that embodiments can be provided in many alternate forms and should not be construed as limited to the examples set forth herein.

Accordingly, while embodiments can be modified in various ways and take on various alternative forms, specific embodiments thereof are shown in the drawings and described in detail below as examples. There is no intent to limit to the particular forms disclosed. On the contrary, all modifications, equivalents, and alternatives falling within the scope of the appended claims should be included. Elements of the example embodiments are consistently denoted by the same reference numerals throughout the drawings and detailed description where appropriate.

The terminology used herein to describe embodiments is not intended to limit the scope. The articles "a," "an," and "the" are singular in that they have a single referent, however the use of the singular form in the present document should not preclude the presence of more than one referent. In other words, elements referred to in the singular can number one or more, unless the context clearly indicates otherwise. It will be further understood that the terms "comprises," "comprising," "includes," and/or "including," when used herein, specify the presence of stated features, items, steps, operations, elements, and/or components, but do not preclude the presence or addition of one or more other features, items, steps, operations, elements, components, and/or groups thereof.

Unless otherwise defined, all terms (including technical and scientific terms) used herein are to be interpreted as is customary in the art. It will be further understood that terms in common usage should also be interpreted as is customary in the relevant art and not in an idealized or overly formal sense unless expressly so defined herein.

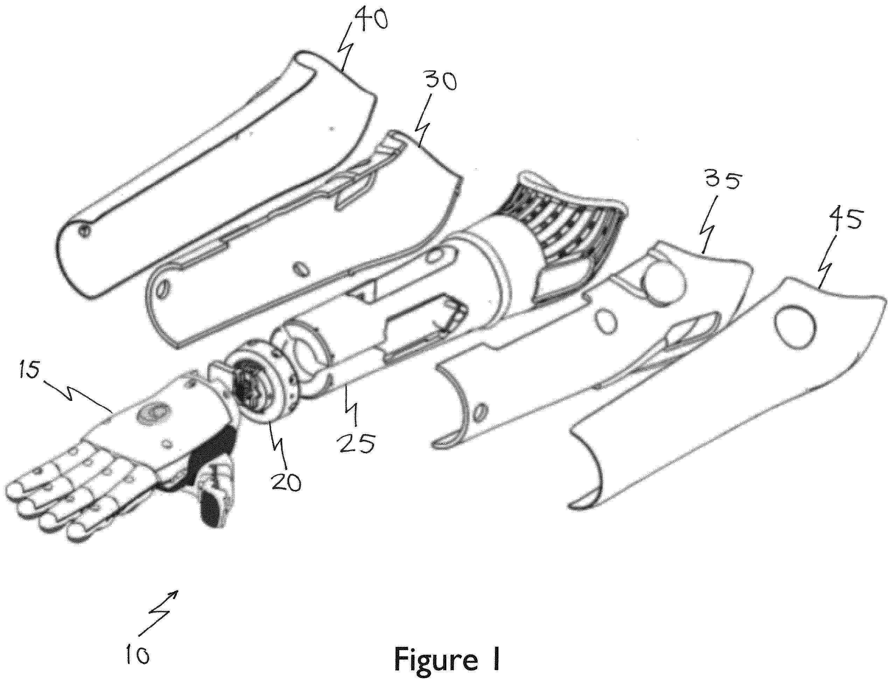

Referring first to Figure 1, there is shown a prosthetic arm 10 designed to fit transradial amputees. It is comprised of three main sub-assemblies; the hand 15; the wrist 20; and the socket 25. An outer frame is provided by two frame portions 30, 35. Additionally, optional, swappable covers 40, 45 can be added to style the arm and are detachably attachable to the outer frame portions.

As discussed below, the system is actuated by motors concealed within the palm. It is powered by a battery located, for example, either just below the elbow or inside the distal end of the arm. The user controls the system by flexing the muscles of their forearm; the system senses these flexes with Electromyographic (EMG) sensors embedded in the socket 25.

The arm 10 is designed to offer amputees a level of functionality close to more advanced devices such as the BeBionic v3 from Otto Bock and the i-Limb from Touch Bionics, whilst still being affordable.

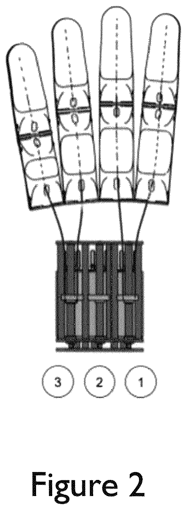

Figure 2 shows a palmar view of the tendon layout for a three motor variant of the actuator block. The central motor is linked to the thumb (this is omitted from this diagram).

The hand contains the actuators and the main control PCB. Although this places a large proportion of the mass far from the elbow, it means the hand can be fitted to a wide range of transradial amputees. Any hardware placed between the end of the user's residual limb and the wrist limits the range of residual limbs that can be fitted. Amputees with an intact wrist would have a disproportionately long prosthetic arm.

In this embodiment the humanoid hand has four fingers and a thumb. It comes in left and right variants, and a variety of sizes.

A smaller size variant uses a three motor actuator block. In this arrangement, the outer two motors are used to flex the fingers by pulling on a tendon.

A full description of the two actuator block variants is given below. In this arrangement, the outer two motors are used to flex the fingers. They do so by pulling on a tendon, a description of this mechanism can be found below.

Motor one flexes the first and second fingers, motor two flexes the thumb, and motor three flexes the third and fourth fingers.

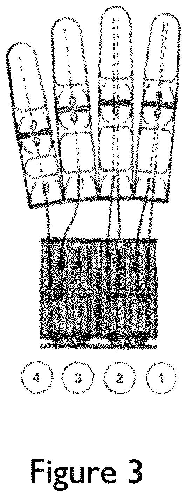

Figure 3 shows a palmar view of the tendon layout for a four motor variant of the actuator block. The third motor is linked to the thumb, this is omitted from the diagram.

For larger hands, there is space to fit a four motor variant of the actuator block. In this case, the first and second fingers are actuated independently. Motor one flexes the first finger, motor two the second finger, motor three is linked to the thumb, and motor four flexes the third and fourth fingers. The arrangement is shown in Figure 3.

In this manner, hands with the four motor variant are capable of more dexterous grip patterns such as pinching.

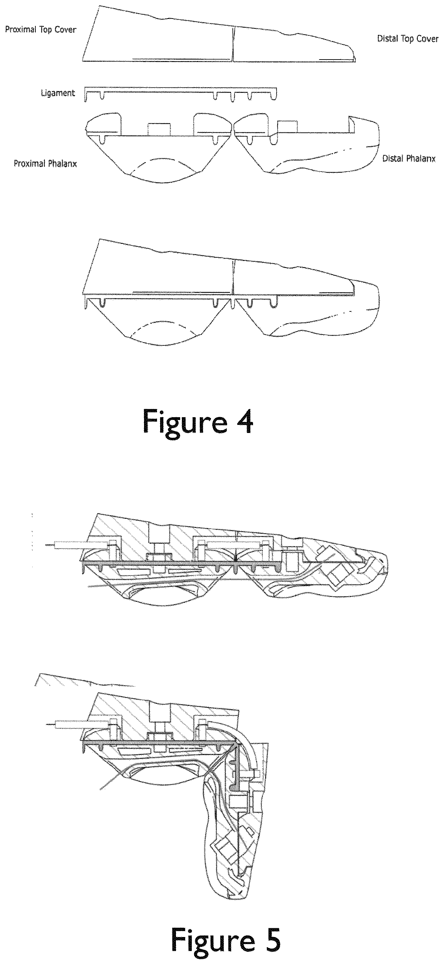

The fingers consist of a flexible "ligament" sandwiched between rigid phalanges and covers. An exploded view is shown in Figure 4.

The flexible ligament acts as a 'living hinge' between the rigid sections formed by the phalanges and covers. This form of joint was chosen because of its high durability. Due to their position on the distal end of the system, the fingers are likely to be the point of contact in any accidental collisions. They are also vulnerable to lateral forces as they are long compared to the width of their base; essentially a lever effect generates high forces at the joints.

The rubber 'living hinge' affords some flexibility, reducing the peak loads experienced in an impact. This also has a knock-on benefit for weight and weight-distribution. Less material is needed to reinforce each finger joint. In this embodiment the ligament is printed from Cheetah, an extremely abrasion resistant TPU. It is the same material the socket is made from.

### Actuation

The fingers are flexed by means of tendons that run through tendon channels in the phalanges.

As the tendons are on the palmar side of the joints, the effect of the actuators retracting the tendons is to flex the fingers - Figure 5.

The actuators in the hand produce linear motion through a lead screw. They therefore have a limited amount of travel. Therefore, the tendon channels have to be carefully positioned so that the full travel of the motor is used.

### Extension Springs

As the fingers are linked to the actuators via a flexible tendon, they are only flexed by the tendon retraction, and cannot extend via pushing of the tendon. To compensate for this, the fingers have a series of springs on the dorsal side which allows them to extend when the actuators relax the tendon. The springs have a slight pre-tension on them when the fingers are fully extended which holds them against their backstops. This prevents the fingers flexing under their own momentum as the user moves the hand around. Without the springs, users report a "floppy" feel to the fingers.

### Tendon Fastening

As shown in Figure 4 and Figure 5, the tendons are fastened at the fingertips. The tendons need to be the right length for each finger; otherwise, some of the travel would be used taking in slack, or the finger would not be able to fully extend. The tendons are also under extremely high loads. Due to the leverage involved in a tendon system, the tension in the tendon can be an order of magnitude higher than the grip force exerted by a fingertip. As such, the tendon fastening mechanism needs to be both adjustable and capable of withstanding high loads. It also needs to be compact enough to fit in the fingertip. It is also extremely hard to get the tendon length correct.

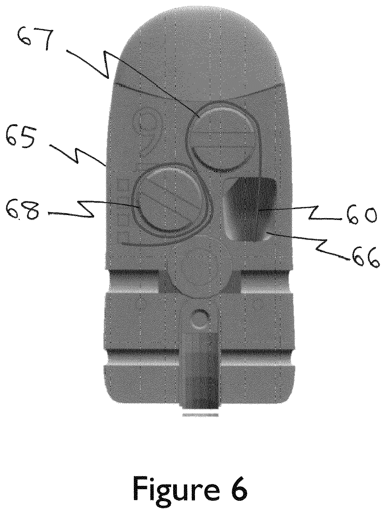

Figure 6 shows a tendon fastening system used in some embodiments. The tendon 60 exits the fingertip 65 from a channel outlet port 66. Two large-headed bolts 67, 68 are provided. The tendon can be wound around the bolts in a figure of eight configuration as shown. The tendon can be slid through to change the effective working length. To secure the tendon the bolt heads are screwed down.

### Thumb

The thumb tendon exits through a hole in the thumb mounting point. A consequence of this is that the thumb motor is reversed compared to the finger motors. The nut moves distally (towards the fingers) to oppose the thumb.

### Palm

The palm of the hand contains the control PCB, and the actuators.

The central component of the hand is the chassis. It is the main load bearing component of the palm. It also provides attachment points for the finger ligament, knuckle, the actuator and PCB block, the thumb and the wrist.

The main load path through the hand goes from the fingers, through the chassis, and finally the wrist. The chassis and wrist dovetail together to provide a strong connection, which is reinforced by two M2 bolts. The wrist and chassis are printed as separate parts to ensure the layer lines are oriented favourably for both components.

### Internal Layout

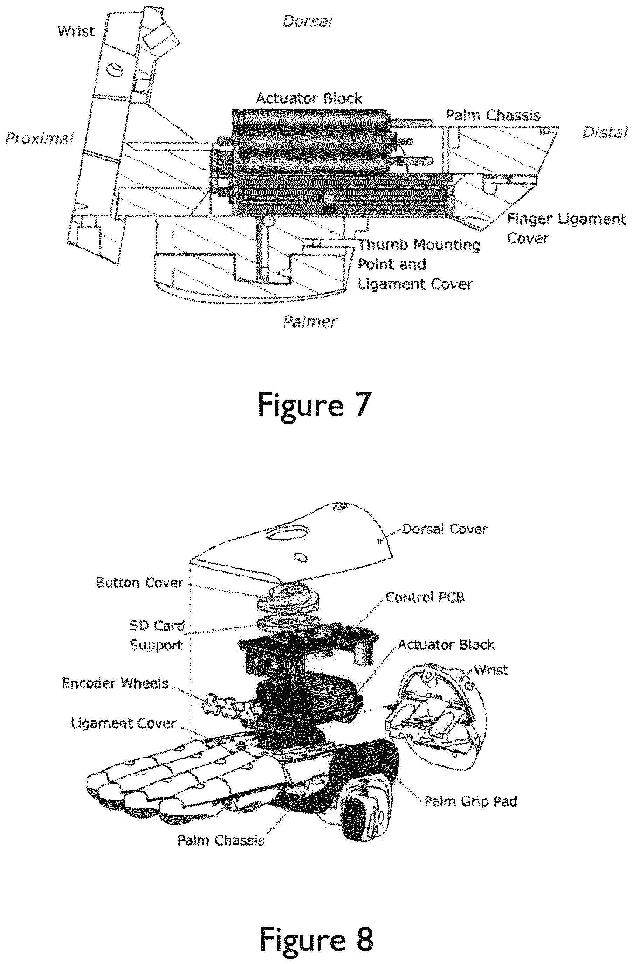

In this embodiment the actuators are comprised of DCX12L motors and a custom gearbox, both provided by Maxon Motor. The gearbox steps down the motor output and converts it from rotational movement to a linear movement via a lead screw. The motor is situated above the lead screw as shown in Figure 7.

This arrangement allows the nut (tendon tensioner) on the lead screw to travel the full distance of the unit. This maximises the energy (i.e. force x distance) available to flex the fingers.

The position of the nuts is measured by rotary encoders attached to the distal end of the motors. As encoders measure movement, rather than the absolute position of the nuts on the lead screws, the ability to detect when the nuts are at the ends of the lead screw is required. This is done by slowly extending the motors until the speed of encoder clicks drops off as the nut hits the compression springs at the end of its travel. The springs also prevent high shock loads in the case of a nut hitting its endstop.

The PCB is designed to fit around the motors as compactly as possible, and to fit in the natural shape of the hand. As such, all the tall components are along the central axis of the dorsal side of the board, with the exception of the two large power capacitors that are on the palmar side behind the motors. The rest of the palmar side has low profile parts allowing it to fit as close to the motors as possible.

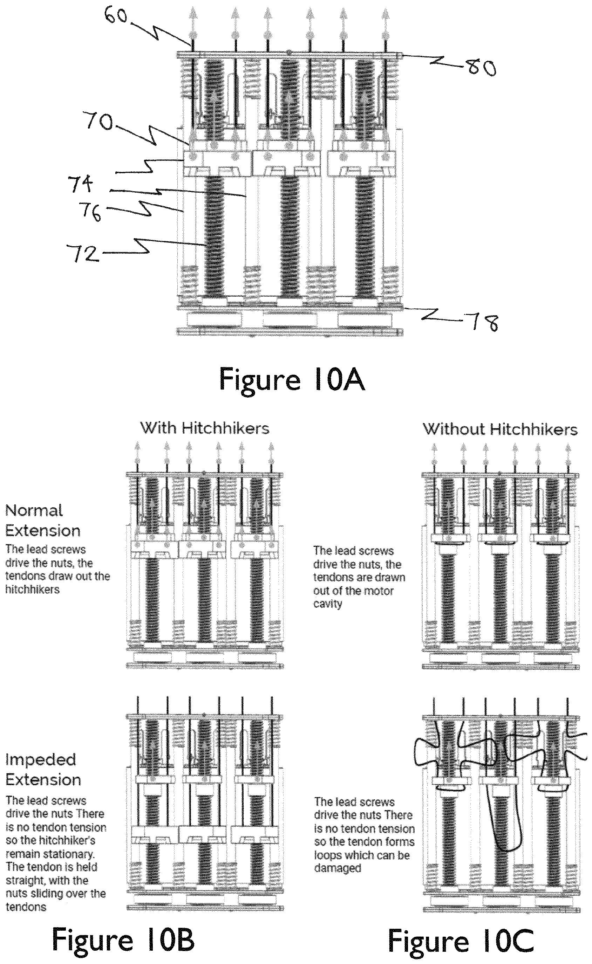

Figure 10A illustrates the functionality of the actuator block.

The motors work through a gearbox to move drive nuts backwards and forwards along lead screws.

Each tendon 60 is connected to a drive nut 70 (e.g. the nut has two holes and the tendon is threaded through the holes) so the tendon moves with the nut. The nut is threaded onto a lead screw 72. Running parallel to and either side of the lead screw is a guide rod 74, 76. The nut is prevented from rotating by the guide rods, along which the nut can slide. This means that when the lead screw 72 is rotated by the motor, depending on which way the screw is rotated, the nut moves towards the proximal end 78 or the distal end 80 of the block.

When the nut moves towards the proximal end this pulls on the tendon and the finger will flex. When the nut moves towards the distal end this relaxes the tendon and the springs cause the finger to straighten (i.e. the motors do not power finger extension).

In normal operation the tendon will move backwards and forwards with the nut, with no slack in the tendon. However, as illustrated in Figure 10C, if extension of the finger is impeded (e.g. if the user tries to straighten the finger but cannot, for example because they are holding it closed, or if the fingers are prevented from straightening as they are held on a surface) this can create a problem. For example, if the finger is fully flexed, with the nut at or towards the proximal ends, and then the user tries to straighten the finger (but cannot) there is no tendon tension; this causes slack in the tendon.

To solve this problem the present invention conceives of the use of "hitchhikers" 74. The hitchhikers 74 are not attached to the lead screw, but they can slide along the guide rods 74, 76. The tendon is also looped through the hitchhikers.

In normal operation the hitchhiker is pushed proximally by the nut and is pulled by the tendon distally, so that it follows the nut. For example as the finger is bent the nut is moved proximally by the motor and the hitchhiker is pushed proximally by the nut. As the finger straightens the nut is moved distally by the motor and the hitchhiker is pulled by the tendon (and is allowed to move along behind the nut).

In the case where the finger cannot straighten the hitchhiker stays where it is even though the nut is being moved by the motor. There is no tendon tension so the hitchhiker remains stationary, preventing slack in the tendon. When the finger is eventually released there is tension back on the tendon and the hitchhiker is pulled back onto the proximal side of the nut.

Figures 10B and 10C illustrates the functionality of the actuator block and also a "hitchhiker" system used in some aspects and embodiments of the present invention.

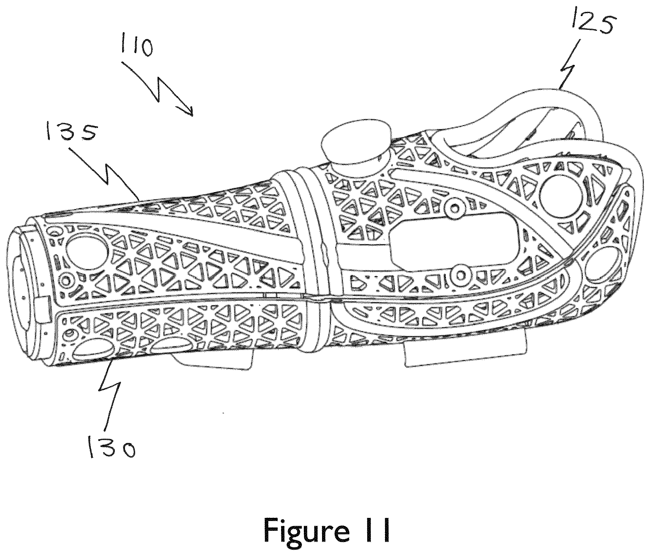

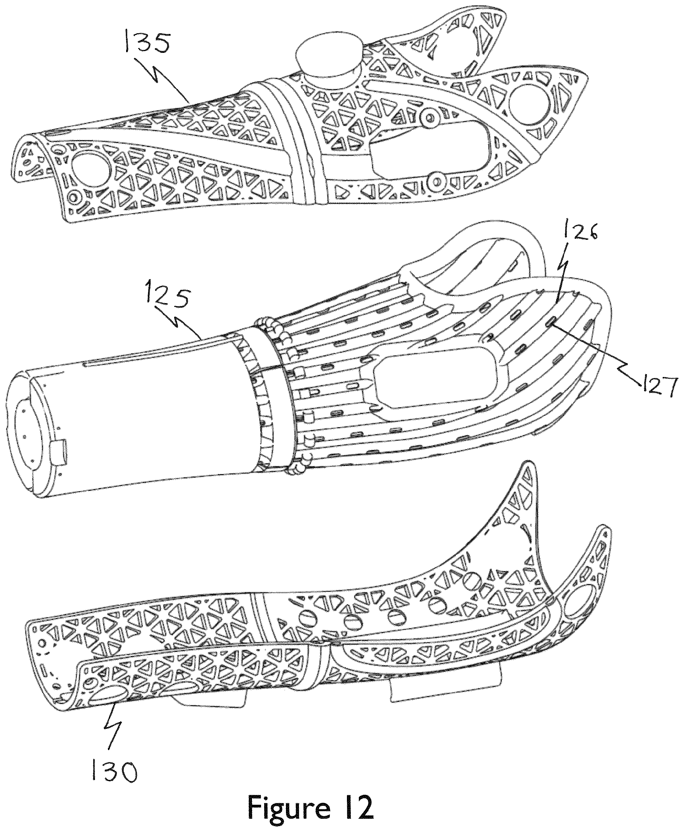

Figures 11 and 12 show various features on a prosthetic arm 10 formed in accordance with the present invention. The socket 125 is shown and external to the socket are two large frames 130, 135 that provide an adjustable clamping force to retain the socket on the arm.

The fingers described above may be used in conjunction with a prosthetic hand as described herein and also with the arm described here.

Ventilation of the socket is achieved due to the fluted nature of the socket where small air channels have been incorporated, the fluted channels 126 are printed with holes 127 to allow heat and moisture to escape externally and allow fresh air to permeate through to the skin.

The covering frames are also aerated via a mesh like structure which helps reduce heat containment.

The covering frames can compress on the socket flutes via a tensioning system and due to the thin walled nature of the flutes, the socket adjusts its diameter to conform to a range of shapes.

Although illustrative embodiments of the invention have been disclosed in detail herein, with reference to the accompanying drawings, it is understood that the invention is not limited to the precise embodiments shown and that various changes and modifications can be effected therein by one skilled in the art without departing from the scope of the invention as defined by the appended claims and their equivalents.

## Claims

1. An actuator for a prosthetic digit, the actuator comprising a driven, mobile tendon tensioner to which a tendon can be attached, the tensioner being movable along a shaft in either direction so as to place tension on the tendon or to relax the tendon.

2. An actuator as claimed in claim 1, in which the shaft comprises a lead screw that can be rotated in either direction.

3. An actuator as claimed in claim 1, further comprising one or more guide rods for preventing rotation of the tensioner as it moves along the shaft.

4. An actuator as claimed in claim 1, in which the shaft can be rotated by a motor.

5. An actuator as claimed in claim 1, further comprising a hitchhiker member to which a tendon is attached but which is not connected to the shaft.

6. An actuator as claimed in claim 5, in which the hitchhiker prevents slack in the tendon.

7. An actuator as claimed in claim 6, in which the hitchhiker prevents slack in the tendon as tensioner moves from a more tensioned position to a less tensioned position.

8. An actuator block comprising one or more actuators as claimed in claim 1.

9. A prosthetic limb comprising one or more actuators as claimed in claim 1.
